# EUROPEAN PATENT SPECIFICATION

(11) **EP 2 566 950 B1**
(45) Date of publication and mention of the grant of the patent: **06.09.2017**
(21) Application number: 11720647.4
(22) Date of filing: 02.05.2011
(51) Int. Cl.: C12M 1/36, C12M 3/00

(54) **METHOD AND APPARATUS FOR CONTROLLING A CELL EXPANSION APPARATUS**
VERFAHREN UND VORRICHTUNG ZUR STEUERUNG EINER ZELLEXPANSIONSVORRICHTUNG
PROCÉDÉ ET APPAREIL POUR COMMANDER UN DISPOSITIF DE DÉVELOPPEMENT CELLULAIRE

(30) Priority: 04.05.2010 US 331249 P
(43) Date of publication of application: 13.03.2013
(73) Proprietor: Terumo BCT, Inc., Lakewood, CO 80215 (US)
(72) Inventor: ANTWILER, Glen Delbert, Lakewood, Colorado 80215 (US)
(74) Representative: J A Kemp
(86) International application number: PCT/US2011/034764
(87) International publication number: WO 2011/139957

(56) References cited:
- US-A- 6 077 708
- US-A1- 2010 042 260
- CLAUSEN A ET AL: "LACTATE AS AN INDICATOR OF TERMINATING TIME IN INSECT CELL CULTURE BACULOVIRUNS EXPRESSION VECTOR SYSTEMS", BIOTECHNOLOGY TECHNIQUES, CHAPMAN & HALL, vol. 10, no. 10, 1 October 1996 (1996-10-01), pages 721-726, XP002588206, ISSN: 0951-208X
- OZTURK S S ET AL: "Real-Time Monitoring and Control of Glucose and Lactate Concentrations in a Mammalian Cell Perfusion Reactor", BIOTECHNOLOGY AND BIOENGINEERING, WILEY & SONS, HOBOKEN, NJ, US, vol. 53, no. 4, 1 February 1997 (1997-02-01), pages 372-378, XP002588207, ISSN: 0006-3592

## Description

The present invention is directed toward a method and apparatus for controlling an apparatus for cell expansion. The cell expansion apparatus or bioreactor system comprises a closed loop cell expansion region where selected cells grow and multiply and a nutrient supply region that provides oxygen and nutrients to sustain the growing cells. The two regions are separated by a membrane. The present apparatus and method controls the bioreactor system by determining volume and rate of cell growth in the cell expansion region by monitoring the rate of nutrient use and the rate of change of the rate of nutrient use in the nutrient supply region.

Stem cells can be expanded from a few donor cells in a cell expansion apparatus. The resulting multiplied cells can be used to repair or replace damaged or defective tissues. Stem cells have broad clinical applications for a wide range of diseases. Recent advances in the area of regenerative medicine have demonstrated that stem cells have unique properties such as high proliferation rates and self-renewal capacity, ability to maintain an unspecialized cellular state, and the ability to differentiate into specialized cells under particular conditions.

As an important component of regenerative medicine, bioreactor systems play an important role in providing optimized environments for cell expansion. The bioreactor provides efficient nutrient supply to the cells and removal of metabolites, as well as furnishing a physiochemical environment conducive to cell growth. In particular, foreign cells, such as airborne pathogens, must be excluded from the cell-growth areas of the bioreactor. At the same time it is important to be able to determine how much cellular growth has taken place. Apparatus and methods for hermetically sampling expanding cellular material from cell-growth areas of a bioreactor, without environmental contamination, such as the apparatus and method of US Application No. 12/021,013, "Disposable Tubing Set for Use with a Cell Expansion Apparatus and Method for Sterile Sampling." Sensors in tubing of a bioreactor have been used to sense lactate levels as a predictor of the number of cells within the bioreactor, as disclosed in US Patent Application 12/536,707, "A Predictor of When to Harvest Cells Grown in a Bioreactor".

The present apparatus and method controls a bioreactor system by determining volume and rate of cell growth in the cell expansion region by monitoring the rate of nutrient use and the rate of change of the rate of nutrient use in the nutrient supply region. Rate of lactate creation and rate of change of rate of lactate creation may be used instead of or in conjunction with nutrient change. An in-line biosensor continuously senses conditions in selected lines, such as a waste line, to determine outflow conditions. In-flow conditions may be determined from pre-determined fluid concentrations and operation of pumps and valves. Preferably, out flow conditions are held relatively constant with regard to nutrient or lactate levels by feedback from a sensor in an outflow path through a computer controller, which adjusts the operation of pumps controlling in-flow. The pump rate or rates will have a changing speed and acceleration proportional to expected changes in nutrient level or lactate level, in the absence of added fluid. Thus, the speed and acceleration of the pump or pumps can be used as a surrogate for changes in the cell population in the bioreactor system. The number of cells in the bioreactor system can be derived from the speed of the pumps. The time needed to achieve growth to a selected number of cells may be calculated from the acceleration of the pumps.

The bioreactor system comprises a disposable apparatus for cell expansion, having at least one bioreactor. The bioreactor has a cellular growth area and a nutrient supply area, the cellular growth area being separated from the supply area by a membrane. The membrane inhibits migration of cells from the cellular growth area to the supply area and permits migration of certain chemical compounds from the cellular growth area to the supply area and of certain other chemical compounds from the supply area to the cellular growth area. At least one oxygenator is in fluid communication with the supply area, and a plurality of bags is in fluid communication with the cellular growth area, the bags providing fluids to the cellular growth area. A fluid recirculation path is in fluid communication with the cellular growth area.

Another aspect of the invention comprises a method of expanding cellular matter, the method comprising providing at least one bioreactor, the bioreactor having a cellular growth area and a supply area, and the cellular growth area being separated from the supply area by a membrane, the membrane being adapted to inhibit migration of cells from said cellular growth area to said supply area and to permit migration of certain chemical compounds from said cellular growth area to said supply area and of certain other chemical compounds from said supply area to said cellular growth area. The method further comprises conducting a fluid containing cellular matter into the cellular growth area; providing oxygenated fluid to said supply area to maintain conditions conducive for cell growth in said cellular growth area; monitoring inflow and outflow conditions; determining rates of change (first derivative) for selected conditions and calculating cell numbers therefrom; determining a rate of change of a rate of change (second derivative) for the selected conditions and calculating a predicted process time; and terminating the growth process when adequate cell numbers have been produced.

The method for controlling a cell expansion apparatus may comprise conducting a fluid containing cellular matter into a cellular growth area; providing fluid to said cellular growth area to maintain conditions conducive for cell growth in said cellular growth area; monitoring inflow fluid conditions to the cellular growth area; monitoring fluid conditions in said cellular growth area; determining rates of change for at least one selected condition and calculating cell numbers therefrom; determining a rate of change of a rate of change for the at least one selected condition and calculating a predicted process time; and terminating the growth process when adequate cell numbers have been produced.

The method may also comprise monitoring a pump rate for providing fluid to the cellular growth area to measure a glucose level or a lactate level. The glucose level or the lactate level may be measured in a waste line. In another aspect of the invention, the glucose level or the lactate level may be maintained at a substantially constant level by controlling the rate fluid is provided to the cellular growth area.

In a further aspect of the invention the rate fluid is provided to the cellular growth area may be controlled by controlling the speed of at least one pump. The rate of change of the rate of change may comprise determining the acceleration of the at least one pump.

These and other features and advantages of the present invention will be apparent from following detailed description, taken with reference to the attached drawings and claims.
FIG. 1 is a schematic description of a cell expansion apparatus.
FIG. 2 is a flow chart of a program for controlling the cell expansion apparatus.
FIG. 3 is a graph representing lactate levels.
FIG. 4 is a graph representing glucose levels.
FIG. 5 is a graph of output lactate and glucose levels during a procedure with increasing levels of input media addition.
FIG. 6 is a graph of input media addition in connection with the procedure of FIG. 5.
FIG. 7 is a flow chart of a second program for controlling the cell expansion apparatus.
FIG. 8 a chart representing lactate and glucose levels for a procedure.
FIG. 9 is a chart of lactate generation and glucose consumption for the procedure of FIG. 8.
FIG. 10 is a chart of intracorporeal fluid (IC) added to the cell expansion apparatus in the procedure of FIG. 8 and FIG. 9.

In the following description of the invention and in the accompanying drawings, like numerals refer to like parts. A bioreactor system **10** comprises a cell expansion module **12** coupled to an intracorporeal (IC) media bag **22** for replenishing fluids and an oxygenator **14** coupled to an extracorporeal (EC) media bag **16** for providing oxygen and removing waste.

### Cell expansion module

The cell expansion module **12,** or bioreactor, may be comprised of semi-permeable hollow fibers or flat sheet membranes enclosed in a housing. If hollow fibers are used, the fibers may be made of a biocompatible polymeric material such as Polyamix, which is a blend of polyamide, polyarylethersulfone and polyvinylpyrrolidone. Depending upon the type of cells to be expanded in the bioreactor, the fibers may or may not be treated with a substance to enhance cell growth and/or adherence to the membrane. The fibers may be held in place within the housing with polyethylene potting. The bioreactor housing has at least four openings into the interior of the housing. Two open into the intra-capillary or IC space, fluidly connecting to the interior of the hollow fibers, and two open into the extracapillary or EC space, fluidly connecting to the space surrounding the hollow fibers.

Cells may be grown in the IC space. The IC space with its minimum volume reduces the quantity of expensive media and expensive cytokines/growth factors required. The semi-permeable membrane allows transfer of metabolic components, waste and gases between the EC and IC compartments. The molecular transfer characteristics of the hollow fibers are chosen to minimize loss of expensive reagents from the IC side, while allowing metabolic waste products to diffuse through the membrane into the EC side to be removed. The EC space carries nutrients to the cells in the IC space, removes waste byproducts and maintains gas balance. The bioreactor may be attached to the rest of the disposable set with connectors made of polyurethane (Tygothane C-210-A).

### Oxygenator

The oxygenator **14** used may be any commercially available oxygenator. One possible oxygenator is manufactured by Gambro GmbH, Hechingen, Germany. The oxygenator may have a fiber count of 1820, an internal fiber diameter of 280 µm, an outer fiber diameter of 386 µm and an intercapillary fluid volume of 16 mL. The hollow fibers of the oxygenator are enclosed in a housing having four port openings. Inlet **20** and outlet **46** ports are fluidly connected to the interior (intercapillary or IC space) of the hollow fibers. Another set of inlet **48** and outlet **50** ports are fluidly connected to the space surrounding the hollow fibers (extracapillary or EC space).

Through the IC inlet port **20** of the oxygenator, an EC inlet line **18** is connected to deliver either fresh media from the EC media bag **16** or recirculated EC media to the oxygenator **14.** Connected to the IC outlet port **46,** the EC line **18** delivers oxygenated EC media to the EC inlet port **42** on the bioreactor **12.** Connected to the EC inlet port **48** is a line **52** to a source of gas **54** (gas line). The EC outlet port **50** is open to the atmosphere, with a 0.22 µ in-line filter **56** to prevent microbes from entering and contaminating the closed system.

### EC media bag and EC media inlet line

An EC media bag **16,** which contains the media, which will flow through the EC side of the bioreactor, may be connected via a portion of flexible tubing (the EC inlet line) **18** to the IC inlet port **20** of the oxygenator **14.** The EC inlet line **18** brings fresh EC media to the oxygenator **14** to be oxygenated. The EC inlet line **18** may be made of polyvinyl chloride with fluorinated ethylene propylene (PVC/FEP (sold as Tygon SE-200)).

### IC media bag and IC media inlet line

An IC media bag **22** that contains the media that will flow through the IC side of the bioreactor may be connected via a portion of flexible tubing (the IC inlet line) **24** to the IC inlet port **26** of the bioreactor **12.** The IC inlet line **24** brings fresh IC media to the IC side of the bioreactor. The IC inlet line **24** may also be made of PVC/FEP.

### Vent bag

A vent bag **28** may be connected to the disposable set via flexible tubing **27** to collect any air initially in the system, before the system is filled with media and cells.

### Cell input bag

A cell input bag **30** contains the cells to be added to the bioreactor **12.** The cell input bag **30** is connected to the IC inlet line **24,** which delivers cells into the lumen of the hollow fibers via cell input line **29.**

### Cell harvest bag

When the cells are ready to be harvested, they are flushed out of the IC outlet port **34** of bioreactor **12** through cell harvest line **31** and into a cell harvest bag **32.**

### IC Recirculation/reseeding tubing loop

The disposable tubing set also may include a length of tubing which acts as an IC circulation loop **36.** The IC media flows out of the bioreactor **12** from the IC outlet port **34** through tubing loop **36** and back into the bioreactor through the IC inlet port **26.** This loop **36** is used to recirculate the IC media though the hollow fibers. It may also be used to flush the cells out of the hollow fibers and reseed/redistribute them throughout the hollow fibers for further growth.

The IC recirculation loop **36** may contain a sample tube **38,** for example, an additional length of tubing. This additional tubing enables small pieces of the tubing to be sterilely removed from the disposable set and the media inside tested for markers of cellular metabolism such as pH, glucose, lactate, electrolytes, oxygen and carbon dioxide content. The sample tubing **38** may be made of SANIPURE™ tubing (SEBS). The sample tube **38** may be solvent bonded into the IC loop **36** using cyclohexanone.

### EC recirculation loop

An EC recirculation loop **40** allows the media on the EC side of the bioreactor to be recirculated. The EC recirculation loop **40** allows EC media to flow out of the bioreactor from the EC outlet port **42** back into the bioreactor through the EC inlet port **44.** This loop may be used to recirculate the EC media that surrounds the hollow fibers.

### Waste bag

IC and EC media containing metabolic breakdown products from cell growth are removed from the system via tubing **58** into a waste bag **60.**

### Pump loops

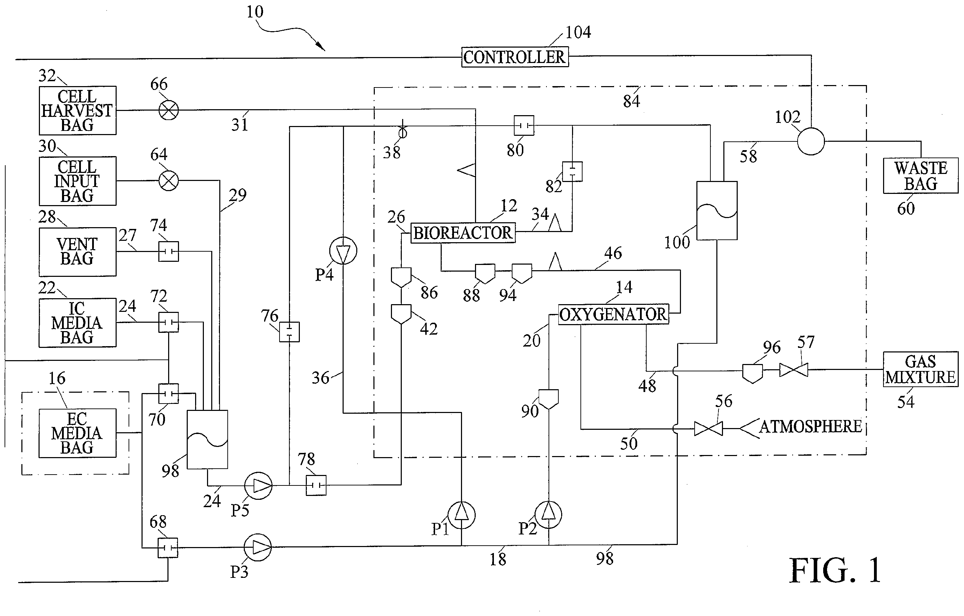

As shown in FIG. 1, the tubing set may engage three or more pump loops that correspond to the location of peristaltic pumps on the cell expansion apparatus. In an embodiment, the tubing set may have five pump loops, corresponding to pumps **P1-P5** on the apparatus. The pump loops may be made of polyurethane (PU (available as Tygothane C-210A)).

### Cassette

A cassette for organizing the tubing lines and which may also contain tubing loops for the peristaltic pumps may also be included as part of the disposable. Additional tubing lines (see **62)** can be added as needed to enable specific applications such as reseeding/redistributing cells in the bioreactor. In order to control the passage of fluid through the disposable **10,** manually operated clamps **64, 66** may be provided. In addition, microprocessor-controlled pinch valves **68, 70, 72, 74, 76, 80, 82** may be coupled to selected tubes of the disposable. Microprocessor-controlled pinch valves are available on blood processing devices such as the TRIMA^{®} apheresis machine, available commercially from the assignee of this invention. A TRIMA^{®} apheresis machine may be modified to accept the disposable **10** by removing a centrifuge ordinarily mounted within the TRIMA^{®} apheresis machine and placing the bioreactor and oxygenator within the machine as an incubator **81.** Temperature sensors **86, 88, 90** and pressure sensors **92, 94,** and **96** can be connected to selected tubes of the disposable **10** and placed in electrical communication with a microprocessor. It is to be understood that pumps, temperature sensors, pressure sensors and pinch valves are preferably connected to the disposable set only temporarily by contact. Manual clamps, on the other hand, are usually mounted on their respective tubes and may be delivered with the disposable.

With the disposable apparatus **10** mounted in the incubator **81,** extracorporeal media is flowed throughout the apparatus **10,** including all connecting tubes, first and second drip chambers **98, 100,** the oxygenator **14** and the bioreactor **12.** Pumps **P1, P2, P3** and **P4,** controlled by the incubator **18** may be selectively activated to force fluid into sections of the disposable apparatus to prime the apparatus. After priming, intercellular media and cells, for example mesenchymal stem cells may be added from bags **22, 30** through the first drip chamber **98** and conducted into a cell expansion area of the bioreactor **14** and related tubing including recirculation path **36** and sample means **38.** The hermetically sealed condition of the apparatus **10** is maintained by providing a vent bag **28** coupled to the first drip chamber **98** to accommodate variations in flow from the EC media bag **16,** the IC media bag **22** and the cell input bag **30.** Driven by pump **P2,** extra corporeal fluid passes through the oxygenator **14** where the fluid is infused with gas into a supply area of the bioreactor **12.** The supply area is separated from the cell expansion area by a membrane that allows oxygen and other desirable chemical components to pass into the cell expansion area and allows waste products of the cell expansion process to pass by osmosis out of the cell expansion area while preventing cellular matter from crossing the membrane. The status of the fluid flowing through the supply area of the bioreactor is monitored by temperature sensor **88** and pressure sensor **94** as well as temperature sensor **90,** which monitors the temperature of the fluid entering the oxygenator **14.** An appropriate gas, such as oxygen, or a gas mixture is conducted through the oxygenator **14** at a pressure monitored by sensor **96.** The gas is preferably medical grade and is also isolated from ambient air by 0.22 micron filters **56, 57.** The characteristics of the extracorporeal fluid can also be checked by withdrawing fluid samples through sample ports **S1** and **S2** on the inflow and outflow lines of the bioreactor. The sample ports have internal filters that allow fluid to be extracted by cellular sized particles from passing into or out of the apparatus **10.**

The pumps are preferably peristaltic pumps. In addition to the manual clamps and automatically controlled valves, the pumps also act as valves, preventing flow of fluid past the pump when the pump is not actively driven. Therefore, when pump **P1** is not in operation and valves **76, 78** are closed, a recirculation loop is formed through the bioreactor 12 and pump **P4.** Conditions in this recirculation loop, where cells are growing, are monitored with temperature sensor **86** and pressure sensor **92** and by taking fluid samples through a sample port **S3.** The fluid extracted through the sample port **S3,** as explained above, does not contain cells or particles of cellular size. It is important to be able to monitor the progress of cellular growth over time without compromising the hermetically sealed conditions of the apparatus **10.** Once the desired cell concentrations have been obtained, the contents of the bioreactor **12** can be harvested into the cell harvest bag **32.**

### Cell Concentration Monitoring

During an expansion cycle, some amount of fluid is typically removed from the fluid circulation paths (the IC and/or EC circuit) at various times throughout the cell expansion cycle and analyzed for the amount of metabolites and other by-products of cell growth in the fluids. The fluid removed from the fluid flow circuits may be run through any commercially available blood gas analyzer (the blood gas analyzer used in this instance was a Siemens 800 series) to measure the amounts of metabolites contained in the fluid. Using a blood gas analyzer, the concentration of lactate (or glucose) is measured in mM/L. Other methods of measurement such as direct chemistry may also be used.

Metabolites may also be measured using a biosensor. Any commercially available biosensor may be used. If the biosensor is sterile, or is made of a material which may be sterilized with ethylene oxide or gamma irradiation, it may be fluidly connected directly into the fluid lines (in-line). If the biosensor is not able to be sterilized, it may be indirectly connected into the fluid lines via a sterile barrier filter. Glucose and lactate molecules are small enough that they diffuse equally across the membrane, and are in equilibrium. Therefore, accurate measurements can be taken by any means on either the IC or EC side, or in waste line **58.** Fluid may be removed from the IC loop **202** through sampling port **216** or sample coil **218** and/or from the EC loop **204** through sample port **230.**

Aerobically growing cells consume glucose and oxygen and produce lactate. The more cells that are present in a cell growth chamber, the more glucose and oxygen are consumed and lactate generated. When cells are at a high density, particularly adherent cells, cell expansion slows due to increased cell-cell interaction between colonies. Cell clumping or aggregation also occurs at high cell density. It is currently not routine practice to look directly inside a cell growth chamber to see if cells are growing into each other without destroying the sterility of the system. Therefore, it would be advantageous if metabolic products of cell growth such as lactate, or products consumed during cell growth such as glucose and oxygen could be used as an indirect measurement to determine if cells were reaching confluence and should be harvested.

An algorithm to determine the number of cell doublings, which, in turn, determines the best time to reseed or harvest the cells before cell growth slows has been disclosed in US Application 12/536,707. The number of doublings can be determined using lactate mass generated and the number of cells initially loaded into the cell expansion system.

The present apparatus and method controls a bioreactor system by determining volume and rate of cell growth in the cell expansion region by monitoring the rate of nutrient use, such as glucose, and the rate of change of the rate of nutrient use in the nutrient supply region. Rate of lactate creation and rate of change of rate of lactate creation may be used instead of or in conjunction with nutrient change. An in-line biosensor **102** may continuously sense conditions in selected lines, such as the waste line **58,** to determine outflow conditions. A suitable sensor for detecting glucose or lactate concentration is available from Jobst Technologies GmbH, Freiburg, Germany. Either a glucose sensor or a lactate sensor or both could be used. In-flow conditions for glucose concentrations and volumes may be determined from pre-determined fluid concentrations and operation of pump **P5** and valve **72.**

As shown in FIG. 1, a digital computer **104** is receives data from the sensor **102** and controls valves and pumps thereby regulating the addition of fluids into and out of the bioreactor. The digital computer **104** may include memory, at least one processor, and a user interface for receiving instructions from a user via a user input device (mouse, keyboard, keypad, touch screen, optical sensor or verbal command). In addition, the digital computer **104** comprises a CES controller interface for relaying information to and from elements such as sensors (pressure, temperature, and biosensor) and for instructing various mechanical systems such as the pumps and valves. The digital computer **104** may be in communication with additional sensors for monitoring other aspects of the apparatus, such as whether one or more fluid bags are low and/or empty. Programming utilized by the digital computer **104** may comprise, by way of example and not limitation, software or firmware.

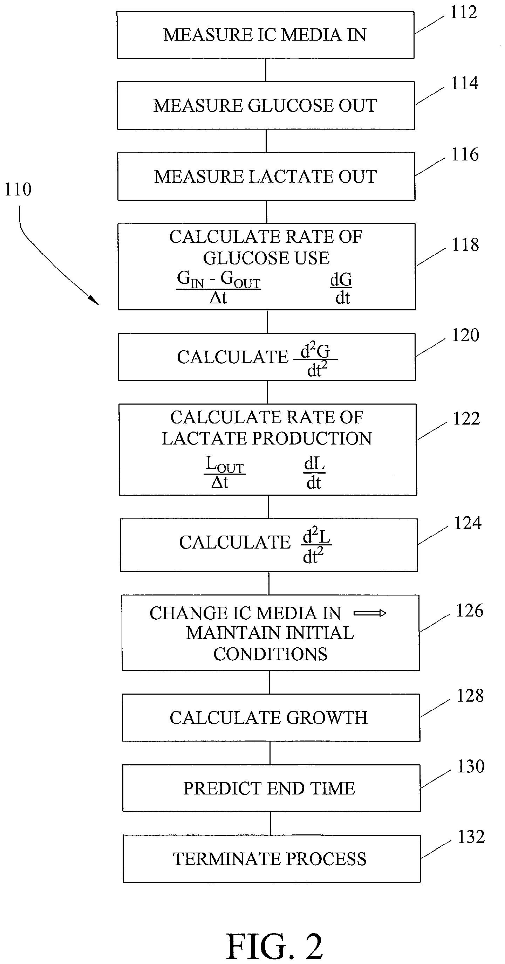

FIG. 2 represents an algorithm **110** for controlling the bioreactor system **10,** and, in particular, for predicting a time for process completion from the rate of change of the rate of change of a selected parameter (the second derivative or acceleration), such as glucose level or lactate level or pump speeds.

Change of glucose in the system may be determined by measuring **112** the volume of solution delivered to the system from the IC media bag **22** or the EC media bag **16.** The volume of solution delivered to the system may be determined from the revolutions of a peristaltic pump coupled to an inflow line containing glucose solution. The contents of the media bags **16, 22** should have a known concentration of glucose solution. Alternatively, one or more sensors could be provided to detect the glucose concentration at the media bags. Glucose removed from the system may be measured **114** by sensing both glucose concentration and fluid volume of fluids being removed to the waste bag **60** past sensor **102.** Multiple measurements correlated with a timer, which may be integrated into the controller or digital computer **104,** allow both the rate of change of glucose concentration (first derivative or "velocity" of change) **118** and the rate of change of the velocity of change (second derivative of "acceleration" of change) **120** to be calculated.

Lactate levels may also be measured **116,** either in conjunction with the measurement of glucose levels, or as an alternative. Since lactate is a product of the cells in the system, the initial condition is that there should be essentially no lactate in the system. Like glucose, lactate removed from the system may be determined by sensing both lactate concentration and fluid volume of fluids being removed to the waste bag **60** past sensor **102.** Multiple measurements correlated with the timer allow both the rate of change of lactate concentration (first derivative or "velocity" of change) **122** and the rate of change of the velocity of change (second derivative of "acceleration" of change) **124** to be calculated.

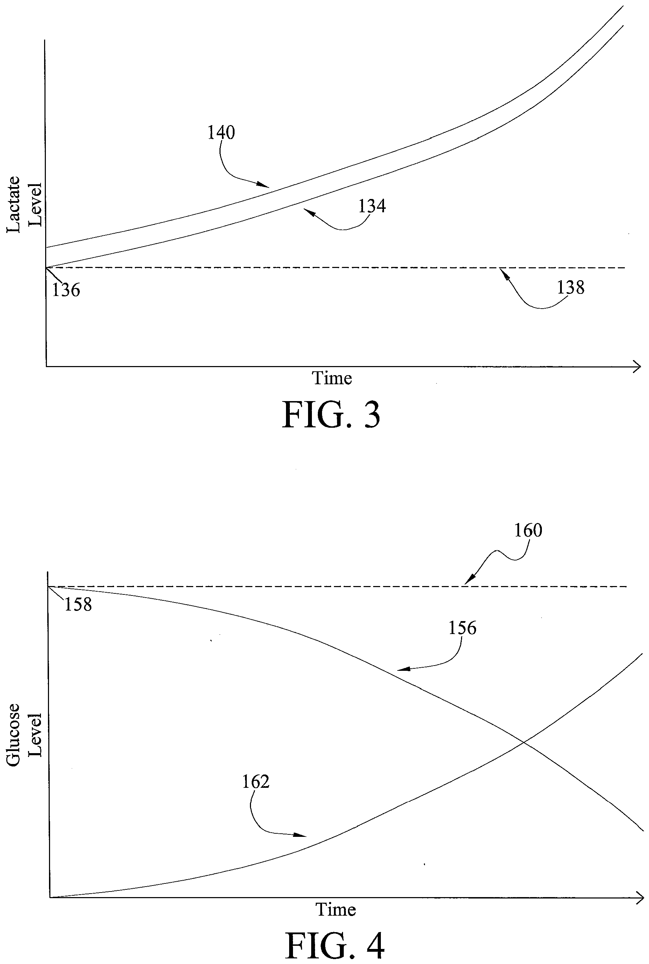

In order to encourage optimum growth, the bioreactor system **10** tries to maintain an initial or optimum growth condition. This may be approximated by periodically adding nutrient fluid to the system and by removing an equivalent amount of fluid from the system, less losses from leakage or evaporation. This process is illustrated in FIG. 3 and FIG. 4. FIG. 3 illustrates two curves **134, 140.** A smooth, continuously rising curve **134** represents increasing lactate level with increasing time at the waste line output sensor **102** or elsewhere within the system, if there were no additional fluid added or removed from the system. In the absence of changing conditions such as adding new fluid, the concentration of lactate would be expected to rise from an initial condition **136** (low or near zero lactate) as a function of the increased number of cells and accumulation of lactate in the system. Of course, if glucose and other nutrients are not added to the system and if wastes are not removed, lactate accumulation will slow or stop. For purposes of this illustration, however, the bioreactor system **10** does add nutrients and remove waste. The actual concentration of lactate in the system **10** is illustrated by dotted line **138.** Both dL/dt and d²L/dt² can be calculated from the measurements described above and from the changes in the pump action adding fluid to the system. Fluid addition to the system is represented by the curve **140.** Rate of change of the lactate level (dL/dt) is proportional to the number of cells in the system **10.** The rate of change of the rate of change (acceleration or d²L/dt²) of the lactate level is proportional to the duration of the process until a desired number of cells will have been grown. This can be used to predict an end time for the process on an on-going, updated basis, thereby reducing the need for continuous monitoring of the system by a human operator. As can be seen in FIG. 3, the fluid addition line **140,** which is directly related to the speed of the pump **P5,** is directly proportional to the expected lactate level **134,** when the lactate level is held relatively constant, as shown by line **138.** A similar process may be employed with respect to the glucose level, as illustrated in FIG. 4. In this case, a smooth, continuously falling curve **156** represents decreasing glucose level with increasing time. In the absence of changing conditions, the concentration of glucose will fall from an initial condition **158** as a function of the increased number of cells and consumption of glucose in the system. Of course, if glucose and other nutrients are not added to the system, the glucose will eventually be completely consumed. As before, the bioreactor system **10** does add nutrients and remove waste. The actual concentration of glucose in the system **10** is illustrated by dashed line **160.** The rate of media addition or the pump speed is represented by rising line **162,** which is congruent with line **140** of FIG. 3. The rate of media addition **162** (which is directly proportional to the pump speed), when the glucose level is held constant, is inversely proportional to the expected glucose level if no media were added. Thus, both dG/dt and d²G/dt² can be calculated from the measurements described above and from the action of the pump or pumps adding fluid to the system. Rate of change of the glucose level (dG/dt) is inversely proportional to the number of cells in the system **10.** The rate of change of the rate of change (acceleration or d²G/dt²) of the glucose level is inversely proportional to the duration of the process until a desired number of cells will have been grown. This can be also be used to predict an end time for the process on an on-going, updated basis, thereby reducing the need for continuous monitoring of the system by a human operator.

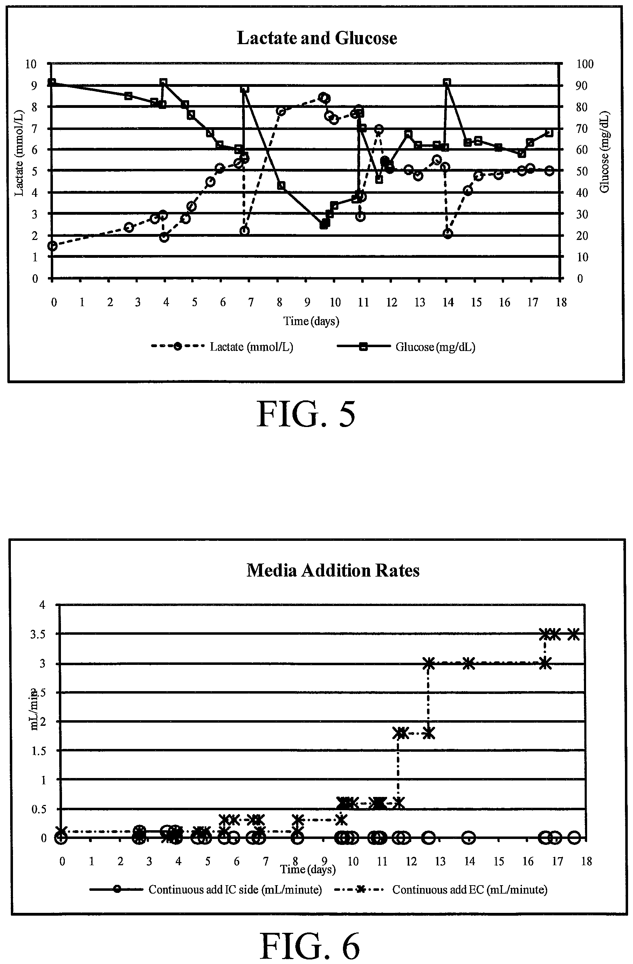

An implementation of this process is illustrated in Figures 5 and 6 wherein amounts of IC fluid and EC fluid were added continuously over a period of eighteen days to a cell growth apparatus. The glucose level was adjusted towards 80 mg/dL. The lactate level was generally held below 9 mmol/L. The levels of lactate and glucose and the lactate generation rate were measured over the 17.62-day growth process, as shown in the following table.

| Table 1. Lactate and Glucose Levels | | | |
|---|---|---|---|
| Time (Days) | Lactate (mmol/L) | Glucose (mg/dL) | Lactate Generation (mmol/day) |
| 0.00 | 1.53 | 91 | 0 |
| 2.70 | 2.36 | 85 | 0.14 |
| 3.66 | 2.8 | 82 | 0.29 |
| 3.95 | 2.93 | 81 | 0.32 |
| 3.96 | 1.93 | 91 | |
| 4.72 | 2.77 | 81 | 0.61 |
| 4.95 | 3.36 | 76 | 1.45 |
| 5.62 | 4.48 | 68 | 1.08 |
| 5.94 | 5.11 | 62 | 2.21 |
| 6.61 | 5.33 | 60 | 1.52 |
| 6.81 | 5.56 | 57 | 2.04 |
| 6.84 | 2.19 | 88 | |
| 8.13 | 7.82 | 43 | 3.58 |
| 9.64 | 8.43 | 25 | 5.44 |
| 9.70 | 8.38 | 26 | 5.02 |
| 9.85 | 7.56 | 30 | 2.05 |
| 10.02 | 7.41 | 34 | 4.23 |
| 10.77 | 7.66 | 37 | 4.90 |
| 10.90 | 7.87 | 39 | 5.77 |
| 10.92 | 2.88 | 77 | |
| 10.99 | 3.8 | 70 | 8.48 |
| 11.61 | 6.95 | 46 | 5.71 |
| 11.79 | 5.44 | 54 | 6.94 |
| 11.95 | 5.12 | 53 | 7.83 |
| 12.65 | 5.04 | 67 | 8.31 |
| 12.97 | 4.8 | 62 | 7.44 |
| 13.64 | 5.5 | 62 | 8.98 |
| 13.94 | 5.19 | 61 | 8.37 |
| 14.01 | 2.1 | 91 | |
| 14.73 | 4.12 | 63 | 7.46 |
| 15.13 | 4.8 | 64 | 12.67 |
| 15.81 | 4.85 | 61 | 13.35 |
| 16.65 | 5.03 | 58 | 13.92 |
| 16.96 | 5.1 | 63 | 17.33 |
| 17.62 | 5.03 | 68 | 17.15 |

As shown in FIG. 6, the rate of EC fluid addition in mL/minute was incrementally increased over the period. Although this graph shows discrete changes in fluid addition rate, it is clear that mathematical methods could be used to fit a curve to the discrete data. Moreover, with the control feedback described herein, a smoother addition rate curve would be developed, and both the lactate and glucose levels shown in FIG. 5 could be held within closer tolerances.

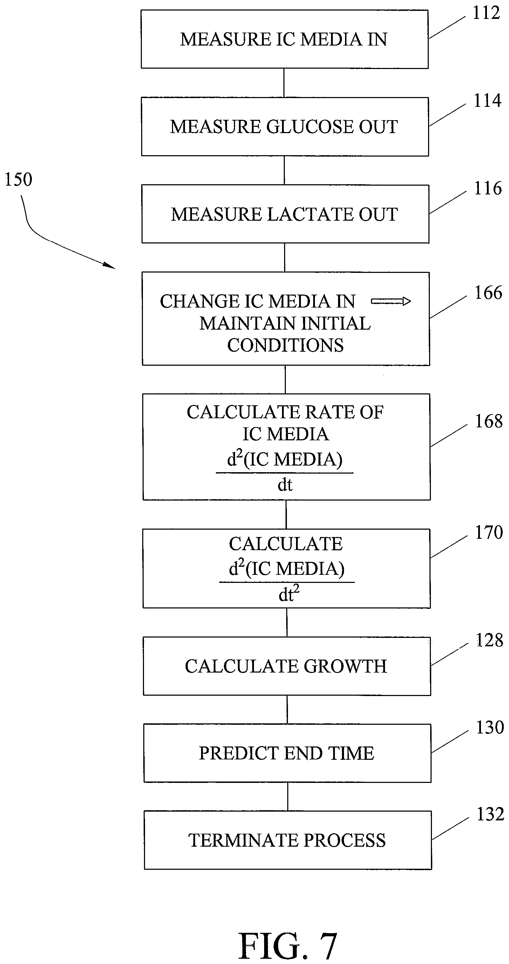

The continuous addition of new solution to the system is also shown in the schematic **150** of FIG. 7. The level of lactate at the waste bag **60** would preferably be substantially constant. Deviations would be corrected **166** by the controller or digital computer **104** increasing or decreasing the rate of a pump **P5** coupled to the IC media bag **22** or the EC media bag **16** through valves **72, 70,** respectively. Once again, the amount of fluid added during the procedure will rise exponentially, preferably continuously. The speed at which fluid is added and the acceleration of fluid addition can be known from the speed and acceleration of the pump or pumps. Either lactate level or glucose level or both could be measured **114, 116** and corrections **166** made in the pump speed, such as acceleration, to maintain the lactate and glucose levels at relatively constant levels. After the corrections, a smoothed curve representing the speed of the pump or pumps may be calculated **168** and a curve representing the acceleration of the speed of the pump or pumps can be calculated **170** and used to further calculate **128** the number of cells in the bioreactor (from the speed of the pump or pumps) and to predict **130** the time at which the desired number of cells will be grown (from the acceleration of the pump or pumps).

In one implementation with continuous IC fluid addition, an initial aliquot of about 20 million mesenchymal stem cells was loaded in a cell expansion system. After a five and a half day growth period, about 184 million cells were harvested. Table 1 records lactate and glucose levels and use over the growth period.

| Table 2. Continuous IC Fluid Addition | | | | |
|---|---|---|---|---|
| Time (days) | Lactate (mmol/L) | Glucose (mg/dL) | Lactate Generation | Glucose Generation |
| 0.00 | 1.69 | 92 | 0.000 | 0.000 |
| 0.65 | 2.22 | 89 | 0.424 | 0.271 |
| 0.81 | 2.12 | 88 | | 0.331 |
| 0.90 | 1.89 | 92 | | |
| 1.63 | 2.56 | 86 | 0.507 | 0.360 |
| 1.93 | 2.79 | 86 | 0.492 | 0.150 |
| 2.64 | 3.67 | 78 | 0.795 | 0.493 |
| 2.96 | 4.15 | 76 | 1.025 | 0.392 |
| 4.02 | 5.04 | 64 | 1.191 | 0.791 |
| 5.16 | 4.64 | 74 | 3.368 | 1.536 |
| 5.64 | 3.44 | 76 | 4.077 | 2.631 |

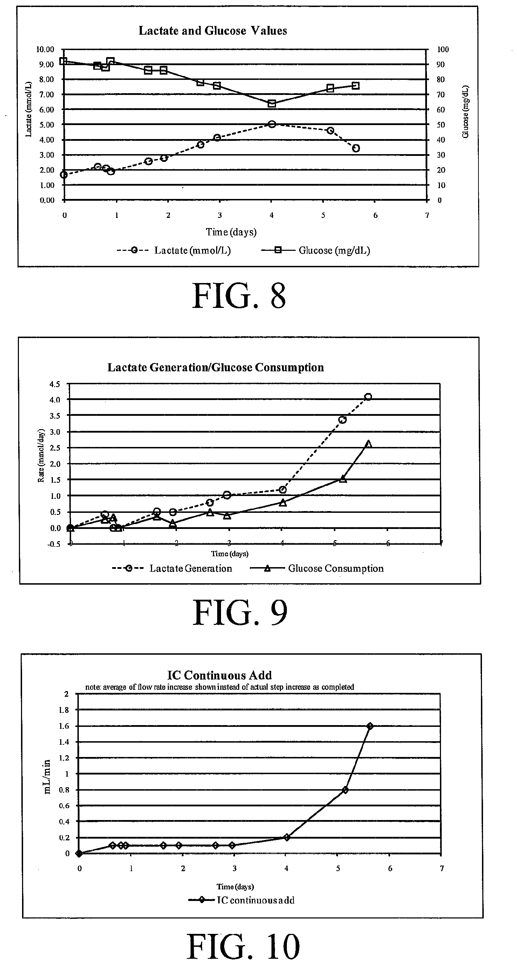

As seen in FIG. 8, the lactose level was held relatively constant with values throughout the growth period between 1.5 mmol/L and 5 mmol/L. At the same time, the glucose level was held relatively constant, falling between about 92 mg/dL and 62 mg/dL. The rates of lactate generation and glucose consumption, illustrated in FIG. 9, showed a similar, increasing trend, to which a curve could be fitted and rate of generation/consumption and acceleration of generation/consumption could be calculated. As described above, this information could be used to estimate the number of cells in the system (from the rate of consumption) and the time needed to grow a desired number of cells (from the acceleration). FIG. 10 shows that the rate of fluid addition, which is known from the pump rates, shows a similar, geometrically increasing shape. The magnitude of the curve at a selected time is proportional to the speed to the pumps, and the slope of the curve is proportional to the acceleration of the pumps. Consequently, the number of cells in the system at a given time can be derived from the speed of the pumps. The time needed for the growth of a selected number of cells can be derived from the acceleration of the pumps. The system would be able to display these values to an operator either continuously or at discrete intervals. This allows improved monitoring of the system and improved prediction of the time when the cells should be harvested.

The foregoing description of the present invention has been presented for purposes of illustration and description. Furthermore, the description is not intended to limit the invention to the form disclosed herein. Consequently, variations and modifications commensurate with the above teachings, and skill and knowledge of the relevant art, are within the scope of the present invention as defined in the claims. The embodiments described hereinabove are further intended to explain best modes known of practicing the invention and to enable others skilled in the art to utilize the invention in such embodiments, or other embodiments and with various modifications required by the particular application(s) or use(s) of the present invention.

## Claims

1. A method for controlling a cell expansion apparatus, said method comprising
conducting a fluid containing cellular matter into a cellular growth area;
providing fluid to said cellular growth area to maintain conditions conducive for cell growth in said cellular growth area;
monitoring inflow fluid conditions to the cellular growth area;
monitoring fluid conditions, said conditions comprising at least one of nutrient levels or lactate levels in said cellular growth area;
determining rates of change for at least one of said nutrient levels or lactate levels and calculating cell numbers therefrom;
determining a rate of change of a rate of change for the at least one level and calculating a predicted process time; and
terminating the growth process when adequate cell numbers have been produced.

2. The method of claim 1 wherein said inflow condition is a pump rate for providing fluid to said cellular growth area.

3. The method of claim 1 wherein said step of monitoring fluid conditions in said cellular growth area comprises measuring a glucose level.

4. The method of claim 1 or 3 wherein said level is measured in a waste line.

5. The method of claim 1 or 3 wherein said level is maintained at a substantially constant level by controlling the rate fluid provided to said cellular growth area.

6. The method of claim 5 wherein the rate fluid is provided to said cellular growth area is controlled by controlling the speed of at least one pump.

7. The method of claim 6 wherein the step of determining the rate of change of the rate of change comprises determining the acceleration of said at least one pump.

8. An apparatus for cell expansion, said apparatus comprising
at least one bioreactor, said bioreactor having a cellular growth area and a supply area, said cellular growth area being separated from said supply area by a membrane,
means for providing fluid to said cellular growth area to maintain conditions conducive for cell growth in said cellular growth area;
means for monitoring inflow fluid conditions to the cellular growth area;
means for monitoring fluid conditions in said cellular growth area, said conditions comprising at least one of nutrient levels or lactate levels;
means for determining rates of change for at least one of said nutrient levels or lactate levels and calculating cell numbers therefrom; and
means for determining a rate of change of a rate of change for the at least one level and calculating a predicted process time.

9. The apparatus of claim 8 further comprising at least one pump and wherein said monitored inflow fluid condition is a pump rate.

10. The apparatus of claim 8 wherein the means for monitoring conditions in said cellular growth area comprises a glucose sensor.

11. The apparatus of claim 8 wherein the means for monitoring conditions in said cellular growth area comprises a lactate sensor.

12. The apparatus of claim 10 or 11 wherein said sensor is coupled to a waste line.

13. The apparatus of claim 10 or 11 further comprising means for maintaining said level at a substantially constant level by controlling the rate that fluid is provided to said cellular growth area.

14. The apparatus of claim 13 wherein the means for maintaining said level comprises means for controlling the speed of at least one pump.

15. The apparatus of claim 14 wherein the means for determining the rate of change of the rate of change comprises means for determining the acceleration of said at least one pump.

## Patentansprüche

1. Verfahren für ein Steuern einer Zellexpansionsvorrichtung, das Verfahren umfassend:
Leiten eines Fluids, das Zellmaterie enthält, in ein Zellwachstumsgebiet;
Bereitstellen von Fluid an das Zellwachstumsgebiet, um Bedingungen aufrechtzuerhalten, die für ein Zellwachstum in dem Zellwachstumsgebiet förderlich sind;
Überwachen von Zuflussfluidbedingungen zu dem Zellwachstumsgebiet;
Überwachen von Fluidbedingungen, die Bedingungen umfassend mindestens eines von Nährstoffpegeln oder Lactatpegeln in dem Zellwachstumsgebiet;
Bestimmen von Änderungsraten für mindestens einen des Nährstoffpegels oder des Lactatpegels und daraus Berechnen von Zellanzahlen;
Bestimmen einer Änderungsrate einer Änderungsrate für den mindestens einen Pegel und Berechnen einer voraussichtlichen Prozesszeit; und
Beenden des Wachstumsprozesses, wenn geeignete Zellanzahlen hergestellt wurden.

2. Verfahren nach Anspruch 1, wobei die Zuflussbedingung eine Pumprate für ein Bereitstellen von Fluid zu dem Zellwachstumsgebiet ist.

3. Verfahren nach Anspruch 1, wobei der Schritt des Überwachens von Fluidbedingungen in dem Zellwachstumsgebiet ein Messen eines Glukosepegels umfasst.

4. Verfahren nach Anspruch 1 oder 3, wobei der Pegel in einer Abflussleitung gemessen wird.

5. Verfahren nach Anspruch 1 oder 3, wobei der Pegel durch ein Steuern der Rate, mit der dem Zellenwachstumsgebiet Fluid bereitgestellt wird, auf einem im Wesentlichen konstanten Pegel gehalten wird.

6. Verfahren nach Anspruch 5, wobei die Rate, mit der dem Zellwachstumsgebiet Fluid bereitgestellt wird, durch ein Steuern der Geschwindigkeit von mindestens einer Pumpe gesteuert wird.

7. Verfahren nach Anspruch 6, wobei der Schritt zu einem Bestimmen der Änderungsrate von der Änderungsrate ein Bestimmen der Beschleunigung der mindestens einen Pumpe umfasst.

8. Zellexpansionsvorrichtung, die Vorrichtung umfassend
mindestens einen Bioreaktor, wobei der Bioreaktor ein Zellwachstumsgebiet und ein Zuführungsgebiet hat, wobei das Zellwachstumsgebiet durch eine Membran von dem Zuführungsgebiet getrennt ist,
Mittel für ein Bereitstellen von Fluid zu dem Zellwachstumsgebiet, um Bedingungen aufrechtzuerhalten, die für ein Zellwachstum in dem Zellwachstumsgebiet förderlich sind;
Mittel für ein Überwachen von Zuflussfluidbedingungen zu dem Zellwachstumsgebiet;
Mittel für ein Überwachen von Fluidbedingungen in dem Zellwachstumsgebiet, die Bedingungen umfassend mindestens einen von Nährstoffpegeln oder Lactatpegeln;
Mittel für ein Bestimmen von Änderungsraten für mindestens einen des Nährstoffpegels oder Lactatpegels und daraus Berechnen von Zellanzahlen; und
Mittel für ein Bestimmen einer Änderungsrate von einer Änderungsrate für den mindestens einen Pegel und Berechnen einer voraussichtlichen Prozesszeit.

9. Vorrichtung nach Anspruch 8, ferner umfassend mindestens eine Pumpe und wobei die überwachte Zuflussfluidbedingung eine Pumprate ist.

10. Vorrichtung nach Anspruch 8, wobei die Mittel für ein Überwachen von Bedingungen in dem Zellwachstumsgebiet einen Glukosesensor umfassen.

11. Vorrichtung nach Anspruch 8, wobei die Mittel für ein Überwachen von Bedingungen in dem Zellwachstumsgebiet einen Lactatsensor umfassen.

12. Vorrichtung nach Anspruch 10 oder 11, wobei der Sensor an eine Abflussleitung gekoppelt ist.

13. Vorrichtung nach Anspruch 10 oder 11, ferner umfassend Mittel für ein Aufrechterhalten des Pegels auf einem im Wesentlichen konstanten Pegel durch ein Steuern der Rate, mit der das Fluid zu dem Zellwachstumsgebiet bereitgestellt wird.

14. Vorrichtung nach Anspruch 13, wobei die Mittel für ein Aufrechterhalten des Pegels Mittel für ein Steuern der Geschwindigkeit der mindestens einen Pumpe umfassen.

15. Vorrichtung nach Anspruch 14, wobei die Mittel für ein Bestimmen der Änderungsrate von der Änderungsrate Mittel für ein Bestimmen der Beschleunigung der mindestens einen Pumpe umfassen.

## Revendications

1. Procédé permettant de commander un dispositif de développement cellulaire, ledit procédé comprenant :
l'apport d'un fluide contenant de la matière cellulaire dans une zone de croissance cellulaire ;
la fourniture de fluide à ladite zone de croissance cellulaire afin de maintenir des conditions favorables à la croissance cellulaire dans ladite zone de croissance cellulaire ;
la surveillance des conditions d'entrée du fluide dans la zone de croissance cellulaire ;
la surveillance de certaines conditions du fluide, lesquelles conditions comprennent au moins les valeurs nutritives ou les valeurs en lactate présentes dans ladite zone de croissance cellulaire ;
la détermination des taux de variation pour au moins lesdites valeurs nutritives ou valeurs en lactate et le calcul du nombre de cellules à partir de ceux-ci ;
la détermination du taux de variation d'un taux de variation pour l'au moins une valeur et le calcul du délai de réalisation escompté ; et
l'achèvement du processus de développement lorsque le nombre adéquat de cellules a été produit.

2. Procédé selon la revendication 1, dans lequel ladite condition d'entrée de fluide est le débit de pompage permettant de fournir du fluide à ladite zone de croissance cellulaire.

3. Procédé selon la revendication 1, dans lequel ladite étape de surveillance de certaines conditions du fluide dans ladite zone de croissance cellulaire comprend la mesure d'une valeur en glucose.

4. Procédé selon la revendication 1 ou 3, dans lequel ladite valeur est mesurée dans une conduite d'évacuation.

5. Procédé selon la revendication 1 ou 3, dans lequel ladite valeur est maintenue à un niveau sensiblement constant en régulant le débit de fluide fourni à ladite zone de croissance cellulaire.

6. Procédé selon la revendication 5, dans lequel le débit auquel le fluide est fourni à ladite zone de croissance cellulaire est régulé grâce à une régulation du régime d'au moins une pompe.

7. Procédé selon la revendication 6, dans lequel l'étape de détermination du taux de variation du taux de variation comprend la détermination de l'accélération de ladite au moins une pompe.

8. Dispositif de développement cellulaire, ledit dispositif comprenant :
au moins un bioréacteur, lequel bioréacteur présente une zone de croissance cellulaire et une zone d'apport, ladite zone de croissance cellulaire étant séparée de ladite zone d'apport par une membrane,
un moyen de fourniture de fluide à ladite zone de croissance cellulaire afin de maintenir des conditions favorables à la croissance cellulaire dans ladite zone de croissance cellulaire ;
un moyen de surveillance des conditions d'entrée de fluide dans la zone de croissance cellulaire ;
un moyen de surveillance de certaines conditions du fluide dans ladite zone de croissance cellulaire, lesdites conditions comprenant au moins des valeurs nutritives ou des valeurs en lactate ;
un moyen de détermination des taux de variation pour au moins lesdites valeurs nutritives ou lesdites valeurs en lactate et le calcul du nombre de cellules à partir de ceux-ci ; et
un moyen de détermination du taux de variation d'un taux de variation pour l'au moins une valeur et le calcul du délai de réalisation escompté.

9. Appareil selon la revendication 8, comprenant en outre au moins une pompe et dans lequel ladite condition d'entrée de fluide surveillée est le débit de la pompe.

10. Appareil selon la revendication 8, dans lequel le moyen de surveillance de certaines conditions dans ladite zone de croissance cellulaire comprend un détecteur de glucose.

11. Appareil selon la revendication 8, dans lequel le moyen de surveillance de certaines conditions dans ladite zone de croissance cellulaire comprend un détecteur de lactate.

12. Appareil selon la revendication 10 ou 11, dans lequel ledit détecteur est couplé à une conduite d'évacuation.

13. Appareil selon la revendication 10 ou 11, comprenant en outre un moyen permettant de maintenir ladite valeur à un niveau sensiblement constant en régulant le débit de fluide fourni à ladite zone de croissance cellulaire.

14. Appareil selon la revendication 13, dans lequel le moyen permettant de maintenir ladite valeur comprend un moyen permettant de réguler le régime d'au moins une pompe.

15. Appareil selon la revendication 14, dans lequel le moyen permettant de déterminer le taux de variation du taux de variation comprend un moyen permettant de déterminer l'accélération de ladite au moins une pompe.
